# EUROPEAN PATENT APPLICATION

(11) **EP 3 090 799 A2**
(43) Date of publication of application: **09.11.2016**
(21) Application number: 15183485.0
(22) Date of filing: 02.09.2015
(51) Int. Cl.: B01J 13/00

(54) **METHOD FOR PREPARING HYDROCOLLOID HAVING DIVERSE RANGE OF HYDROPHOBICITY**

(30) Priority: 06.05.2015 KR 20150063104
(71) Applicant: Soongsil University Research Consortium Techno-Park, Seoul 156-030 (KR)
(72) Inventor: JEONG, Jae Hyun, 120-160 Seoul (KR); RYU, Hee Wook, 137-828 Seoul (KR); KIM, Yun Gon, 122-736 Seoul (KR); KIM, Hee Jin, 426-823 Ansan-si Gyeonggi-do (KR)
(74) Representative: Nordic Patent Service A/S

(57) **Abstract**

The present invention relates to a method for preparing hydrocolloid having a diverse range of hydrophobicity, and more particularly, to a method for preparing hydrocolloid having a diverse range of hydrophobicity including preparing a hydrophobic hydrocolloid molecule by introducing a hydrophobic molecular group to a hydrocolloid molecule, and gelation by adding the hydrophobic hydrocolloid molecule to a pure hydrocolloid molecule.

According to the present invention, a hydrophobic molecule is capable of being stably introduced without decomposing a hydrocolloid molecule during a synthesis process for introducing the hydrocolloid molecule, and hydrocolloid having a diverse hydrophobicity range is capable of being prepared depending on a mixing ratio.

## Description

### TECHNICAL FIELD

The present invention has been made through a project number 10048242 under the support of the Ministry of Trade, Industry and Energy, and the research management specialized agency of the project is the Korea Evaluation Institute of Industrial Technology, the name of the research program is "Royalty-Supported Program", the title of the research project is "Development of agar-based alternative animal testing methods and automatic measurement system for the efficiency and safety-assessment of functional materials.", the leading organization is Foundation of University-Industry Cooperation of Soongsil University, and the research period is from 2014.06.01. to 2015.05.31.

The present invention relates to a method for preparing hydrocolloid having a diverse range of hydrophobicity, and more particularly, to a method for preparing hydrocolloid having a diverse range of hydrophobicity including preparing a hydrophobic hydrocolloid molecule by introducing a hydrophobic molecular group to a hydrocolloid molecule, and gelation by adding the hydrophobic hydrocolloid molecule to a pure hydrocolloid molecule.

### BACKGROUND ART

The global market size for cosmetics has recently increased to approximately 225.7 billion dollars, and the Korean market size is 6.3 billion and 4 million dollars, which corresponds to the 11^{th} largest size in the world. Particularly, the market size of functional cosmetics introduced from 2001 has gradually increased, and the Korean market size of cosmeceuticals is 120 billion won per year and increases by 15% or greater each year.

With consistent pursuit of an FTA system and the like, enhancing global competitiveness in the cosmetics industry is pressing. Securing domestic independent technologies for promoting export and strengthening global competitiveness in the cosmetic industry, one of the 3 biggest industries in Korea, is urgent.

In addition, an income level increase, stress, worse environmental pollution, life extension and the like engaged with a desire of modern people wishing to cherish beauty for a long time have made skin care become an ordinary life style, and as a result, interests in the effectiveness of cosmetics has been rising.

Evaluation on the effectiveness of cosmetics is normally carried out through an artificial skin cell test method, an animal test method and a clinical test method, and the test methods are diversely classified depending on the function of the cosmetics.

In the case of a moisturization effectiveness test, clinical tests are normally carried recently due to a trend opposing animal tests, and a method of measuring moisture content and a method of measuring a transdermal moisture loss content are used. However, skin is an outermost part of our body meeting an external environment, therefore, may sensitively react or change to external environmental changes. Internally, skin is related to diseases and conditions of a body and appears as complex mechanism. Accordingly, skin may be affected by a surrounding environment of a person, the person's living attitudes, diseases and the like even when measuring the skin using an objective device, and therefore, personal differences also need to be considered in order to determine a skin condition with a measuring device. These methods are difficult to set a control group and secure significant data despite the use of expensive devices.

In the case of a transparency test, an evaluation on the sense of transparency, a cognitive effectiveness that has not been possible until now, becomes possible since methods measuring the sense of transparency of a skin or factors affecting the sense of transparency have been identified due to an advance in researches regarding dermal light properties. However, such test methods are normally carried out through clinical tests, and therefore, it has been difficult to secure statistically significant data.

The cosmetics industry domestic and abroad currently focuses on producing an artificial skin capable of replacing animal tests. However, an artificial skin has limits in the application due to enormous production time and costs.

Meanwhile, agar gel is readily purchased, and is currently diversely used mostly as an edible material, an industrial material, a raw material for pharmaceuticals, a microbial solid medium, and a raw material for cosmetics, and therefore, is capable of being developed as various applied products.

In view of the above, researches on preparing agar gel that is simple to use replacing animal tests and has diverse hydrophobicity in order to quickly analyze functionality and safety have been carried out. However, in existing hydrophobic agar gel preparation methods, a hydrophobic molecule may be introduced to an agar gel molecule, however, there has been a problem in that the agar gel molecule is decomposed during a synthesis process. In addition, there has been a problem in that hydrophobicity of the finally prepared agar gel decreases as more hydrophobic molecules substitute agar gel molecules.

Additional aspects of the prior art are disclosed in Korean Patent No. 10-121571 and Korean Patent Application Laid-Open Publication No. 10-2008-0044802.

### DISCLOSURE

### TECHNICAL PROBLEM

In view of the above, an object of the present invention is to provide a method for preparing hydrophobic hydrocolloid, which is capable of preparing hydrocolloid having a diverse hydrophobicity range by stably introducing a hydrophobic molecule without decomposing a hydrocolloid molecule during a synthesis process for introducing the hydrophobic molecule.

In addition, an object of the present invention is to provide a method for preparing colloid having a diverse range of hydrophobicity, which is capable of quickly analyzing functionality and stability of cosmetics using a simple method of use since a test method-based material having properties similar to age-specific, environment-specific and structural skin is capable of being developed.

### TECHNICAL SOLUTION

According to an aspect of the present invention, there is provided a method for preparing hydrocolloid having a diverse range of hydrophobicity, the method including preparing a hydrophobic hydrocolloid molecule by introducing a hydrophobic molecular group to a hydrocolloid molecule; and gelation by adding the hydrophobic hydrocolloid molecule prepared above to a pure hydrocolloid molecule.

The hydrocolloid may be agarose, agar, cellulose, dextran, gelatin, xanthan, carrageenan, alginate, agar gel, pectin, starch, guar, locust bean gum, arabic gum (ARA), carboxymethylcellulose (CMC), gellan gum (GELL), hydroxypropylmethylcellulose (HPMC), sodium alginate (ALG), xanthan gum (XAN) and the like.

The hydrophobic molecular group may be an alkyl chain (C12-18), alkenyl, an acyl chain (C12-18), polypropylene glycol (PPG), poly(caprolactone) (PCL) and the like.

The hydrophobic hydrocolloid molecule may be prepared by introducing the hydrophobic molecular group to the hydrocolloid molecule using an isocyanate synthesis method.

The hydrophobic hydrocolloid molecule preferably has a hydrophobic degree of substitution (DS) of 5% or less.

The hydrophobic hydrocolloid molecule is preferably added in 0.1 to 50% by weight to the total weight of the pure hydrocolloid molecule.

The gelation may be carried out by constant temperature pressurization at 120°C in an aqueous solution. In addition, cooling or ageing may be additionally carried out after the constant temperature pressurization.

According to another aspect of the present invention, the present invention provides hydrocolloid having a diverse range of hydrophobicity prepared using the method described above, which thereby has a hydrophobic degree of substitution (DS) of 5.0 to 50% and a contact angle of 5 to 80 degrees.

It should be understood that different embodiments of the invention, including those described under different aspects of the invention, are meant to be generally applicable to all aspects of the invention. Any embodiment may be combined with any other embodiment unless inappropriate. All examples are illustrative and non-limiting.

### ADVANTAGEOUS EFFECTS

According to the present invention, hydrocolloid having a diverse hydrophobicity range can be prepared by stably introducing a hydrophobic molecule without decomposing a hydrocolloid molecule during a synthesis process for introducing the hydrophobic molecule. In addition, hydrophobic hydrocolloid prepared according to the present invention can be developed as a test method-based material having properties similar to age-specific, environment-specific and structural skin, and can be used as a test material capable of quickly analyzing functionality and stability of cosmetics using a simple method of use.

### BRIEF DESCRIPTION OF THE DRAWINGS

FIG. 1 is a diagram showing the results of measuring the safety of a hydrophobic agar gel molecule prepared by substituting a hydrophobic molecular group to an agar gel molecule by 5% and 20% each according to one embodiment of the present invention.
FIG. 2 is a diagram showing the result of analyzing and identifying the structure of a hydrophobic agar gel molecule prepared by substituting a hydrophobic molecular group to an agar gel molecule by 5% according to one embodiment of the present invention.
FIG. 3 is a diagram showing the result of measuring a contact angle of agar gel prepared differing a degree of substitution of a hydrophobic agar gel molecule according to one embodiment of the present invention.
FIG. 4 is a diagram showing the result of measuring a contact angle of agar gel prepared differing a mixing ratio of a hydrophobic agar gel molecule having a degree of substitution of 5% and a pure agar gel molecule according to one embodiment of the present invention.
FIG. 5 is a diagram showing changes in swelling ratio and an internal structure of agar gel prepared by mixing a hydrophobic agar gel molecule having a degree of substitution of 5% and a pure agar gel molecule according to one embodiment of the present invention.

### BEST MODE FOR THE INVENTION

Exemplary embodiments of the present invention will be described below in more detail with reference to the accompanying drawings. The present invention may, however, be embodied in different forms and should not be construed as limited to the embodiments set forth herein. Rather, these embodiments are provided so that this disclosure will be thorough and complete, and will fully convey the scope of the present invention to those skilled in the art. Throughout the disclosure, like reference numerals refer to like parts throughout the various figures and embodiments of the present invention.

In the present invention, a method of stably introducing a hydrophobic molecule to a hydrocolloid molecule is developed, and hydrocolloid having a diverse hydrophobicity range using the same is prepared in order to overcome a problem of the hydrocolloid itself not having sufficient hydrophobicity even when a hydrophobic molecular group is simply introduced to an existing hydrocolloid molecule.

The present invention relates to a method for preparing hydrocolloid having a diverse hydrophobicity range by introducing a hydrophobic molecular group to a hydrocolloid molecule, and then gelation through adding the hydrocolloid molecule substituted with hydrophobicity to a simple hydrocolloid molecule.

Hereinafter, the method for preparing hydrophobic hydrocolloid of the present invention will be described in more detail.

First, a hydrophobic hydrocolloid molecule is prepared by introducing a hydrophobic molecular group to a hydrocolloid molecule.

As the hydrocolloid, gelation materials dissolved and having viscosity without being melted in a solvent may be used without limit, and examples thereof may include agarose, agar, cellulose, dextran, gelatin, xanthan, carrageenan, alginate, agar gel, pectin, starch, guar, locust bean gum, arabic gum (ARA), carboxymethylcellulose (CMC), gellan gum (GELL), hydroxypropylmethylcellulose (HPMC), sodium alginate (ALG), xanthan gum (XAN) and the like.

As the hydrophobic molecular group, alkyl, alkenyl, acyl chain (C12-18), polypropylene glycol (PPG), poly(caprolactone) (PCL) and the like may be used.

The hydrophobic hydrocolloid molecule may introduce a hydrophobic molecular group to a hydrocolloid molecule using an isocyanate synthesis method. As one example, the following Reaction Formula 1 shows a reaction reforming agar or agarose, one type of a hydrocolloid molecule, to be hydrophobic using an isocyanate synthesis method.

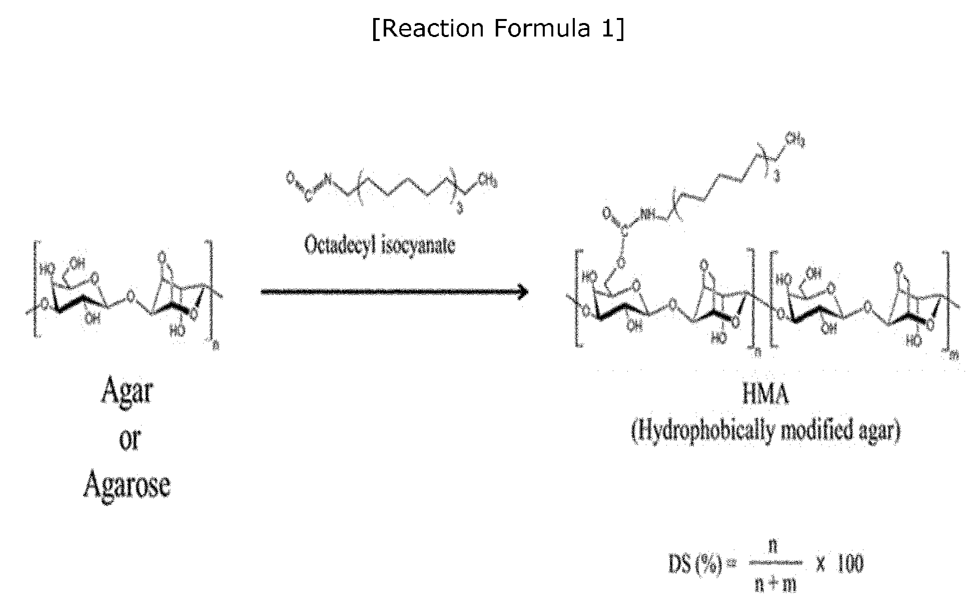

As shown in Reaction Formula 1, an agar or agarose molecule, one type of hydrocolloid, forms hydrophobic molecular group-introduced agar or agarose by octadecyl isocyanate at room temperature, and forms a hydrophobic hydrocolloid molecule of which hydrophobic degree of substitution (DS) is adjusted through the above-mentioned synthesis method.

When a hydrophobic molecular group is introduced to a hydrocolloid molecule using an isocyanate synthesis method as above, a hydrophobic molecule may be stably introduced without a problem of the hydrocolloid molecule being decomposed to small hydrocolloid molecules its physical properties declining during the synthesis.

When a hydrophobic molecular group is simply introduced to an existing hydrocolloid molecule, physical properties such as strength decline due to the decomposition of the hydrocolloid molecule even when a hydrophobic molecule is introduced, and there is a problem in that hydrophobicity of the finally prepared hydrocolloid decreases even when a hydrophobic molecular group largely substitutes the hydrocolloid molecule. However, in the present invention, a hydrophobic molecule may be stably introduced without decomposing a hydrocolloid molecule using an isocyanate synthesis method, and therefore, a hydrophobic molecular group may be introduced to a hydrocolloid molecule with a diverse degree of substitution (DS, 1.0% to 50%).

When a degree of substitution of hydrophobic molecule-introduced hydrophobic hydrocolloid increases, hydrophobic hydrocolloid may be prepared, however, hydrophobicity decreases above a certain degree of substitution. Accordingly, in the present invention, using a hydrophobic hydrocolloid molecule, which is formed from introducing a hydrophobic molecular group as above, having a degree of substitution (DS) of 5.0% or less is more preferable since hydrocolloid having a diverse hydrophobicity range is capable of being prepared as a result.

Next, the hydrophobic hydrocolloid molecule prepared above in an aqueous solution is formed as a gel by being added to a pure hydrocolloid molecule.

As the hydrophobic hydrocolloid molecule, those having a degree of substitution of 5.0% or less are used, and the hydrophobic hydrocolloid molecule is added in 0.1 to 50% by weight with respect to the total weight of the pure hydrocolloid molecule. Herein, when the hydrophobic hydrocolloid molecule is added in less than 0.1% by weight, hydrophobicity may not be expressed in the final hydrocolloid gel, and when added in greater than 50% by weight, transparency of the final hydrocolloid may decline.

The gelation first includes preparing a hydrocolloid molecule to be in a sol state by constant temperature pressurization at 80 to 130°C, preferably at 120°C in an aqueous solution. When the temperature is less than 80°C during the solation, the pure hydrocolloid molecule and the hydrophobic hydrocolloid molecule may be mixed in a non-uniform sol state, and the temperature being greater than 130°C may cause stability decline of the hydrocolloid molecule.

In addition, after the solation, the result is slowly cooled, aged and gelated to prepare the final hydrocolloid.

The hydrocolloid gone through the cooling or ageing experiences physical binding and changes from being in a sol state to a gel state, and hydrophobic hydrocolloid that the present invention aims to prepare is prepared.

In other words, hydrocolloid having a diverse hydrophobicity range may be prepared by adjusting a mixing ratio while mixing a hydrophobic hydrocolloid molecule substituted in a small degree of substitution as above with a pure hydrocolloid molecule in an aqueous solution.

The hydrophobic hydrocolloid of the present invention prepared as above uses a hydrophobic hydrocolloid molecule of which hydrophobic degree of substitution (DS) is 5.0% or less, and therefore, has diverse hydrophobicity with a contact angle range of 5 to 80 degrees. In addition, the hydrophobic hydrocolloid of the present invention may have consistent mechanical properties since the hydrophobic hydrocolloid does not bring changes in a swelling ratios and micro-structures while having a diverse hydrophobicity range.

The hydrophobic hydrocolloid of the present invention has a diverse hydrophobicity range and is capable of being used for cosmetics, microbial culture media, food industries, medicines and the like, and accordingly, may be developed as diverse applied products.

Hereinafter, the present invention will be described in more detail with reference to examples, however, these examples are for illustrative purposes only, and the scope of the present invention is not limited thereto.

### Example 1. Preparation of Hydrophobic Agar Gel

A hydrophobic agar gel molecule was prepared by substituting an agar gel molecule with an octadecyl alkyl chain (C18), a hydrophobic molecule, using an isocyanate method so that a degree of substitution became 5%.

Hydrophobic agar gel was prepared by adding the hydrophobic agar gel molecule having a degree of substitution of 5% prepared above in 5% by weight, 10% by weight and 20% by weight each with respect to the total weight of a pure agar gel molecule in an aqueous solution, then constant temperature pressurizing the result at 120°C, and then cooling and ageing the result at room temperature.

### Example 2. Preparation of Hydrophobic Agar Gel

A hydrophobic agar gel molecule was prepared in the same manner as in Example 1 except that the octadecyl alkyl chain, a hydrophobic molecule, substituted the agar gel molecule so that a degree of substitution became 10%.

### Example 3. Preparation of Hydrophobic Agar Gel

A hydrophobic agar gel molecule was prepared in the same manner as in Example 1 except that the octadecyl alkyl chain, a hydrophobic molecule, substituted the agar gel molecule so that a degree of substitution became 20%.

### Example 4. Preparation of Hydrophobic Alginate

A hydrophobic alginate molecule was prepared by substituting an alginate molecule with an octadecyl alkyl chain, a hydrophobic molecule, using an isocyanate method so that a degree of substitution became 5%. Hydrophobic alginate gel was prepared by mixing the hydrophobic alginate molecule having a degree of substitution of 5% prepared above in 5% by weight, 10% by weight and 20% by weight each with respect to the total weight of a pure alginate molecule in an aqueous solution, and then adding a calcium ion (CaCl₂) thereto.

### Example 5. Preparation of Hydrophobic Agar Gel

A hydrophobic agar gel molecule was prepared in the same manner as in Example 1 except that polypropylene glycol (PPG), a hydrophobic molecule, substituted the agar gel molecule so that a degree of substitution became 5%.

### Test Example 1. Safety When Substituted with Hydrophobic Molecular Group Using Isocyanate Method

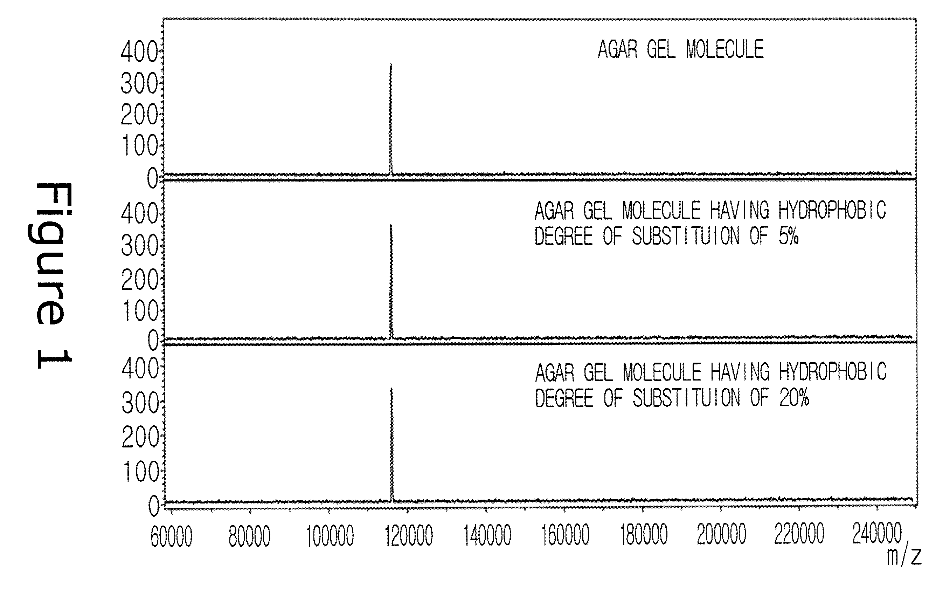

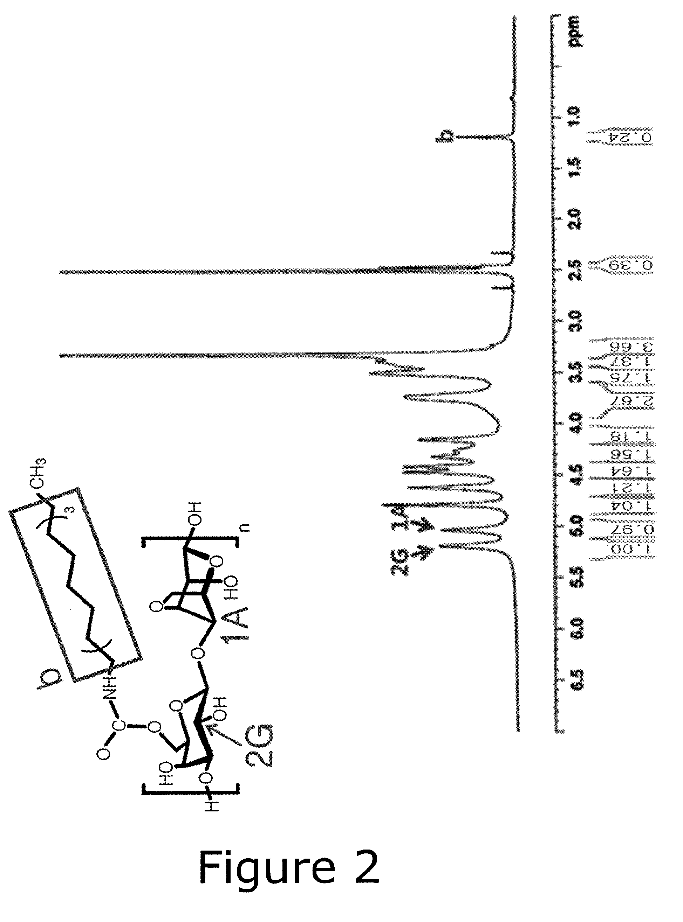

Safety was evaluated through a matrix-assisted laser desorption/ionization (MALDI) analysis method using the agar gel molecule in Examples 1 and 3 substituted by a hydrophobic molecular group in 5% and 20%, respectively, using an isocyanate method, and the results are shown in FIG 1 and FIG. 2.

As shown in FIG. 1, it was identified that, by being substituted with the hydrophobic molecular group in 5% and 20%, the agar gel molecule of Examples 1 and 3 substituted by the hydrophobic molecular group using an isocyanate method according to the present invention was effectively substituted by the hydrophobic molecule as shown in FIG. 2 while the agar gel molecule did not decompose to small molecules.

From the results such as above, it was seen that the hydrophobic molecular group safely substituted without decomposing the hydrocolloid molecule when the hydrophobic molecular group substituted the hydrocolloid molecule using an isocyanate method.

### Test Example 2. Measurement of Contact Angle Depending on Degree of Substitution of Hydrophobic Agar Gel Molecule

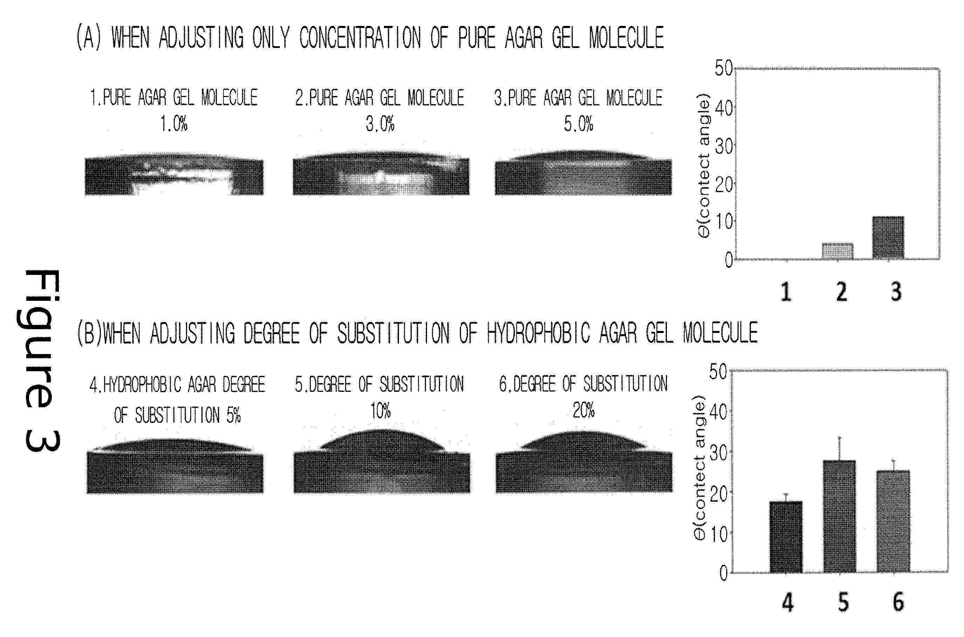

In order to examine a contact angle depending on the degree of substitution of the hydrophobic agar gel molecule, a contact angle of the hydrophobic agar gel prepared using the agar gel molecule substituted with the hydrophobic molecular group in 5%, 10% and 20% each using an isocyanate method in Examples 1 to 3 was measured. Agar gel prepared by adjusting only the concentration of a pure agar gel molecule was employed as a control group, and the results are shown in FIG. 3.

As shown in FIG. 3, there were almost no changes in terms of hydrophobicity depending on the concentration when agar gel was prepared only with the pure agar gel molecule, however, when agar gel was prepared by mixing the pure agar gel molecule and the hydrophobic agar gel molecule prepared by substituting the agar gel molecule with the hydrophobic molecular group according to the present invention, it was identified that the contact angle may be variously adjusted depending on the degree of substitution of the hydrophobic agar gel molecule. However, when the degree of substitution of the hydrophobic molecule increases, it was identified that hydrophobicity decreases above a certain degree of substitution, and therefore, hydrophobic agar gel having a degree of substitution of 5% or less was used in the present invention.

### Test Example 3. Measurement of Contact Angle Depending on Mixing Ratio of Hydrophobic Agar Gel Molecule

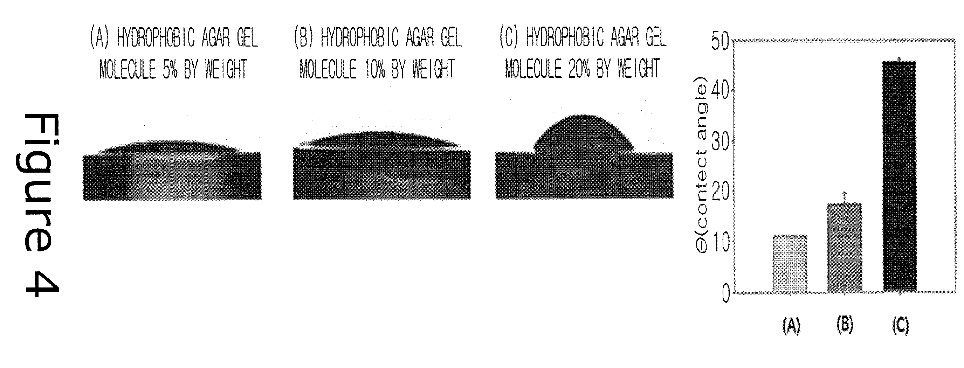

In order to examine a contact angle depending on the mixing ratio of the hydrophobic agar gel molecule, a contact angle of the agar gel prepared by adding the hydrophobic agar gel molecule having a degree of substitution of 5% in 5, 10 and 20% by weight to a pure agar gel molecule in Example 1 was measured, and the results are shown in FIG. 4.

As shown in FIG. 4, when the mixing ratio increases by mixing the hydrophobic agar gel molecule having a degree of substitution of 5% with the pure agar gel molecule, it was identified that agar gel having a diverse hydrophobicity range was capable of being prepared depending on the mixing ratio of the hydrophobic agar gel molecule.

Accordingly, when hydrophobic hydrocolloid was prepared using the hydrophobic hydrocolloid molecule having a degree of substitution of 5% or less according to the present invention, it was seen that hydrocolloid having a diverse hydrophobicity range was capable of being prepared.

### Test Example 4. Swelling Ratio and Micro-structure Changes of Agar Gel

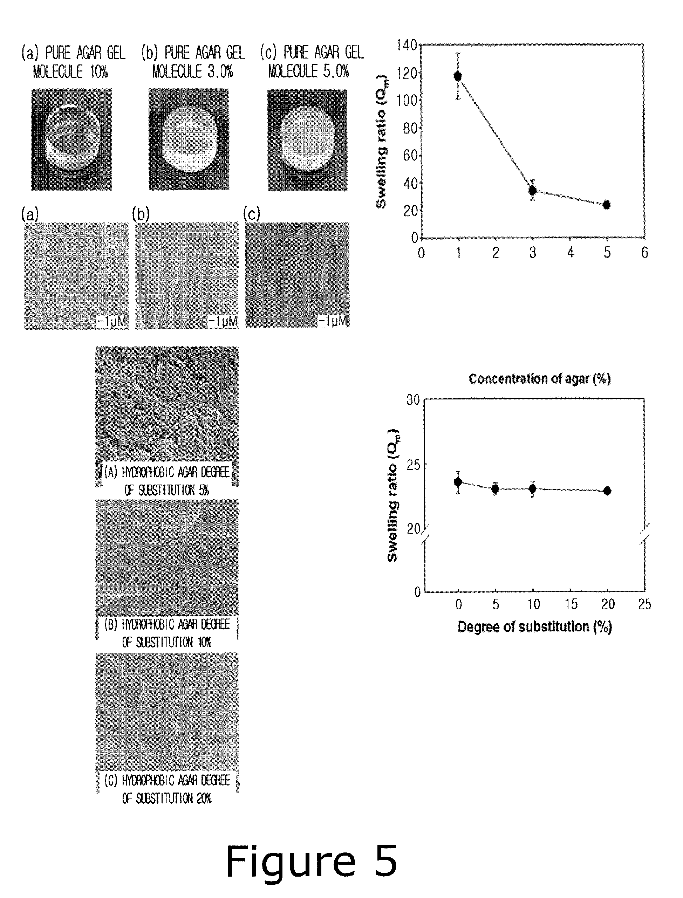

In order to examine a degree of changes in a swelling ratio and micro-structures of the agar gel prepared according to the present invention, a swelling ratio and micro-structures by FE-SEM were measured using the hydrophobic agar gel prepared in Example 1. In addition, agar gel prepared by adjusting only the concentration of a pure agar gel molecule was employed as a control group, and the results are shown in FIG. 5.

As shown in FIG. 5, in the agar gel prepared by mixing the hydrophobic agar gel molecule having a degree of substitution of 5% with the pure agar gel molecule according to the present invention, a swelling ratio and micro-structures did not largely change depending on the mixing ratio of the hydrophobic agar gel molecule, however, when the preparation was carried out using the pure agar gel molecule, it was identified that a swelling ratio and micro-structures changed depending on the concentration of the pure agar gel molecule.

From the result as above, it was able to be predicted that the hydrophobic hydrocolloid prepared according to the present invention was capable of having constant mechanical properties since the hydrophobic hydrocolloid did not cause much changes a swelling ratio and micro-structures while having a diverse hydrophobicity range.

While the present invention has been described with respect to the specific embodiments, it will be apparent to those skilled in the art that various changes and modifications may be made without departing from the spirit and scope of the invention as defined in the following claims.

## Claims

1. A method for preparing a hydrocolloid having a diverse range of hydrophobicity, the method comprising:
preparing a hydrophobic hydrocolloid molecule by introducing a hydrophobic molecular group to a hydrocolloid molecule; and
gelation by adding the hydrophobic hydrocolloid molecule prepared above to a pure hydrocolloid molecule.

2. The method for preparing a hydrophobic hydrocolloid of claim 1, wherein the hydrocolloid is any one or more selected from among agarose, agar, cellulose, dextran, gelatin, xanthan, carrageenan, alginate, agar gel, pectin, starch, guar, locust bean gum, arabic gum (ARA), carboxymethylcellulose (CMC), gellan gum (GELL), hydroxypropylmethylcellulose (HPMC), sodium alginate (ALG) and xanthan gum (XAN).

3. The method for preparing a hydrophobic hydrocolloid of claim 1 or claim 2, wherein the hydrophobic molecular group is any one or more selected from among an alkyl chain (C12-18), alkenyl, an acyl chain (C12-18), polypropylene glycol (PPG) and poly(caprolactone) (PCL).

4. The method for preparing a hydrophobic hydrocolloid according to any of the claims 1 to 3, wherein the hydrophobic hydrocolloid molecule is prepared by introducing the hydrophobic molecular group to the hydrocolloid molecule using an isocyanate synthesis method.

5. The method for preparing a hydrophobic hydrocolloid according to any of the claims 1 to 4, wherein the hydrophobic hydrocolloid molecule has a hydrophobic degree of substitution (DS) of 5% or less.

6. The method for preparing a hydrophobic hydrocolloid according to any of the claims 1 to 5, wherein the hydrophobic hydrocolloid molecule is added in 0.1 to 50% by weight to the total weight of the pure hydrocolloid molecule.

7. The method for preparing a hydrophobic hydrocolloid according to any of the claims 1 to 6, wherein the gelation is carried out by constant temperature pressurization at 120°C in an aqueous solution.

8. The method for preparing a hydrophobic hydrocolloid of claim 7, further comprising cooling or ageing after the constant temperature pressurization.

9. Hydrocolloid having a diverse hydrophobicity range prepared using a method according to any of the claims 1 to 8 and having a contact angle of 5 to 80 degrees.
